(19) Europäisches Patentamt
European Patent Office
Office européen des brevets

(11) **EP 2 515 098 B1**

(12) **EUROPÄISCHE PATENTSCHRIFT**

(45) Veröffentlichungstag und Bekanntmachung des Hinweises auf die Patenterteilung:
**03.04.2019 Patentblatt 2019/14**

(51) Int Cl.:
*G01N 21/31* *(2006.01)*      *G01N 21/35* *(2014.01)*
*G01N 33/00* *(2006.01)*      *G01N 33/14* *(2006.01)*
*G01N 21/3504* *(2014.01)*      *G01N 21/27* *(2006.01)*

(21) Anmeldenummer: **11162834.3**

(22) Anmeldetag: **18.04.2011**

(54) **Verfahren und Vorrichtung zur Bestimmung von SO2 in Nahrungsmitteln und Getränken**

Method and device for determining the SO2 content of food and drink

Procédé et dispositif de détermination de la teneur en SO2 dans des boissons et des aliments

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.10.2012 Patentblatt 2012/43**

(73) Patentinhaber: **Büchi Labortechnik AG**
**9230 Flawil (CH)**

(72) Erfinder: **Egli, Huldrych**
**9200 Gossau (CH)**

(74) Vertreter: **Hepp Wenger Ryffel AG**
**Friedtalweg 5**
**9500 Wil (CH)**

(56) Entgegenhaltungen:
**US-A- 2 736 638    US-A- 2 812 242**

- **Anonymous: "Destillation der gesamten schwefligen Säure nach Dr. REBELEIN", Max F. Keller GmbH , 15. Februar 2005 (2005-02-15), Seiten 1-4, XP002659364, Gefunden im Internet: URL:http://www.keller-mannheim.de/fileadmi n/pdf/getraenke_deutsch/infoblaetter/Schwe flige-S_Best_REBELEIN.pdf [gefunden am 2011-09-19]**
- **SCHWEDT G ET AL: "Comparison of methods (photometry, HPLC, enzymic analysis) for the determination of sulphite in foodstuffs. (translated) TIOL- Methodenvergleiche (Photometrie, HPLC, enzymatische Analyse) zur Bestimmung von Sulfit in Lebensmitteln.", FRESENIUS ZEITSCHRIFT FÜR ANALYTISCHE CHEMIE., Bd. 322, Nr. 3, 1. Januar 1985 (1985-01-01), Seiten 350-353, XP002659365, INST. FUER LEBENSMITTELCHEM., UNIV DOI: 10.1007/BF00490923**
- **MATAIX E ET AL: "Determination of total and free sulfur dioxide in wine by pervaporation-flow injection", ANALYST 1, Bd. 123, Nr. 7, 1998, Seiten 1547-1549, XP002659366, ISSN: 0003-2654, DOI: 10.1039/A802566E**
- **GRANADOS M ET AL: "Determination of sulphur dioxide by flow injection analysis with amperometric detection", ANALYTICA CHIMICA ACTA, Bd. 179, 1. Januar 1986 (1986-01-01), Seiten 445-451, XP026589657, ELSEVIER, AMSTERDAM, NL ISSN: 0003-2670, DOI: 10.1016/S0003-2670(00)84490-6 [gefunden am 1986-01-01]**

**Beschreibung**

Die Erfindung betrifft ein Verfahren sowie eine Vorrichtung zur Bestimmung von Schwefeldioxid ($SO_2$) bzw. schweflige Säure in Getränken und Lebensmitteln, insbesondere Wein und Bier mit den Merkmalen des Oberbegriffs der unabhängigen Patentansprüche. $SO_2$ sowie die im chemischen Gleichgewicht vorliegenden Produkte schweflige Säure, deren Salze und Additionsverbindungen werden im Folgenden unter dem Begriff $SO_2$ zusammengefasst.

Die Schwefelung von Wein mittels $SO_2$ ist eine bekannte Methode zur Konservierung aufgrund der antimikrobiellen Eigenschaften von $SO_2$. Als weitere wichtige Aufgabe wird $SO_2$ aufgrund seiner oxidationshemmenden Eigenschaften verwendet. D.h. oxidative Prozesse mit negativem Einfluss auf die organoleptischen Eigenschaften des Weins werden dadurch unterbunden.

$SO_2$ findet jedoch auch in anderen Getränken wie Bier oder Fruchtsäften oder in anderen Nahrungsmitteln auch fester Konsistenz wie Trockenfrüchten Einsatz als Konservierungsmittel.

Da $SO_2$ aus gesundheitlichen Gründen eine nicht zu vernachlässigende Komponente ist, finden sich in staatlichen Lebensmittelgesetzgebungen Bestimmungen zu maximal erlaubten Konzentrationen. So gilt gemäss der EU-Richtlinie 2003/89/EG eine Deklarationspflicht für Lebensmittel ab einem Gehalt von 10mg/l $SO_2$. Die Symptome für eine $SO_2$-Allergie sind Kopfschmerz und Hautausschlag.

In den Methodensammlungen der Lebensmittelbehörden verschiedener Länder lassen sich unterschiedliche Verfahren zur Bestimmung schwefliger Säure in Wein und anderen Lebensmitteln finden. Zudem schlägt die OIV (Organisation Internationale de la Vigne et du Vin) im "COMPENDIUM OF INTERNATIONAL METHODS OF ANALYSIS" verschiedene Bestimmungsmethoden für schweflige Säure insbesondere in Wein vor.

Die Methode OIV-MA-AS323-04B beschreibt eine iodometrische Bestimmungsmethode, mit einer Titration, wobei der $SO_2$-Gehalt direkt im Wein bestimmt wird. Hierbei stellt sich das Problem, dass die häufig in Wein enthaltene Ascorbinsäure wie schweflige Säure erfasst wird und deshalb gesondert ermittelt und vom erhaltenen Wert abgezogen werden muss. Um diese Mitbestimmung von Ascorbinsäure zu verhindern, bietet es sich an, $SO_2$ aus der Probe zu destillieren.

Die Methode OIV-MA-AS323-04A beschreibt eine Methode zur Bestimmung von $SO_2$ insbesondere in Wein, wobei die schweflige Säure mit einem Stickstoffstrom aus der Probe in ein Auffanggefäß überführt wird und mittels Wasserstoffperoxid oxidiert wird. Die dabei entstandene Schwefelsäure wird anschliessend im selben Gefäss mittels Natronlauge titriert und es wird stöchiometrisch der $SO_2$-Gehalt ermittelt. Die Durchführung dieser Methode mit konventioneller Wasserdampfdestillation von $SO_2$ ist denkbar, besitzt jedoch den Nachteil, dass mit den üblichen Destillationszeiten das $SO_2$ nur unvollständig aus der Probe destilliert und deshalb quantitativ nicht vollständig bestimmt werden kann. In diesem Zusammenhang ergibt sich ein weiterer Nachteil, dass ausreichend lange Destillationszeiten entsprechend grössere Auffanggefässe erfordern.

WO 01/90742 A1 zeigt ein Verfahren und eine Vorrichtung zur Bestimmung von $SO_2$ in einer flüssigen Probe, wobei ausgehend vom gebildeten Gasraum über der Probe das $SO_2$ in der Gasphase gemessen wird. Durch die direkte Bestimmung des $SO_2$-Gehalts soll die Messzeit verkürzt werden. Nach Einstellung eines Gleichgewichts zwischen Gasphase und der flüssigen Probe wird unter Anwendung des Henry'schen Gesetzes aufgrund der Proportionalität zum Partialdruck des Gases die Konzentration des Gases in der (flüssigen) Messprobe ermittelt. Es ist jedoch allgemein bekannt, dass das gelöste Teilchen nicht mit dem Lösungsmittel reagieren darf. Dies ist hier jedoch gerade der Fall, da $SO_2$ mit Wasser zu schwefliger Säure reagiert, was die Genauigkeit der erhaltenen Messwerte anhand dieser Methode einschränkt.

Es ist deshalb eine Aufgabe der vorliegenden Erfindung, ein Verfahren sowie eine Vorrichtung zur Bestimmung von $SO_2$ in Getränken und Lebensmitteln bereitzustellen, welche die beschriebenen Nachteile des Standes der Technik überwinden und welche insbesondere eine einfache, schnelle und präzise Bestimmung des Gehalts an $SO_2$ erlauben.

Erfindungsgemäss werden diese Aufgaben mit einem Verfahren und einer Vorrichtung mit den Merkmalen der unabhängigen Patentansprüche gelöst. $SO_2$ wird zuerst unter Ansäuerung aus der Probe vorzugsweise mittels Destillation, insbesondere Wasserdampfdestillation, aus der Messprobe überführt und in mindestens einem Auffanggefäss aufgefangen. Das Auffanggefäss enthält eine vorbestimmte Menge Iod. Anschliessend erfolgt eine Gehaltsbestimmung, insbesondere titrimetrisch. Vorgängig werden dieselben Verfahrensschritte mit gleichen Verfahrensparametern anhand von Referenzproben mit bekanntem $SO_2$-Gehalt durchgeführt und es wird eine Kalibrationsgleichung ermittelt. Aus den Messwerten der Messproben werden anhand dieser Kalibrationsgleichung die Konzentrationswerte von $SO_2$ in der Messprobe errechnet.

Die Destillationszeit für allfällige Blindwertbestimmungen (siehe weitere Erläuterungen nachfolgend) ist mit derjenigen für die Referenzproben zur Erstellung der Kalibrationsgleichung und für die Messproben identisch.

Es wurde gefunden, dass bei Anwendung des erfindungsgemässen Verfahrens die Messergebnisse einer Probe nach einer verkürzten Destillations- bzw. Messzeit überraschend identische Ergebnisse liefert im Vergleich zu den Messergebnissen aus derselben Probe beispielsweise nach der bekannten Messmethode OIV-MA-AS323-04A, welche jedoch eine bedeutend längere Messzeit erfordert.

Es ist denkbar, die Kalibrationsgleichung im Einzelfall durch Messung von Referenzproben unmittelbar vor der Messung der Probe zu ermitteln. Auch kann diese Kalibrationsgleichung in guter Näherung mehrmals verwendet und gegebenenfalls in regelmässigen Zeitintervallen bestätigt werden. Bevorzugt werden die Kalibrationsgleichungen für definierte Messbedingungen vor Inbetriebnahme auf einem Speicher der erfindungemässen Vorrichtung gespeichert und anschliessend in regelmässigen Intervallen verifiziert, um gute Resultate zu gewährleisten und die Korrektheit der Methode zu bestätigen.

Vorzugsweise ist diese Kalibrationsgleichung eine Korrelationsgleichung, wobei eine Serie von Referenzproben unterschiedlicher, bekannter $SO_2$-Gehalte (y) und die mit dem erfindungsgemässen Verfahren ermittelten $SO_2$-Gehalte (x), insbesondere titrimetrisch, bei identischer Messzeit rechnerisch in Beziehung gebracht werden. Bevorzugt werden die Referenzproben als Volumina oder Einwaagen einer $SO_2$-Standardlösung erhalten.

Es hat sich herausgestellt, dass sich bei definierten Messbedingungen eine reproduzierbare Funktion y=f(x) ermitteln lässt, womit auch Werte zwischen den Messpunkten rechnerisch ermittelbar sind. Dies ist für die Anwendbarkeit der Umrechnung der eigentlichen Messprobe, z.B. eine Weinprobe, wesentlich.

Insbesondere kann die Korrelationsgleichung eine lineare Regressionsgleichung sein, welche anhand einer linearen Regressionsanalyse ermittelt wurde. Es hat sich insbesondere herausgestellt, dass der quadratische Pearson'sche Korrelationskoeffizient der Messpunkte aus den Referenzproben beim Anlegen einer Geraden über die Messpunkte dem idealen Wert von $R^2 = 1$ am nächsten kommt; x und y stehen deshalb in einer linearen Beziehung zueinander.

Eine Ansäuerung der Messproben ist bevorzugt für die Erzielung genauer Messwerte, denn $SO_2$ ist wie bereits erläutert in verschiedenen Zustandsformen in der Probe vorhanden. Deshalb ergibt die Summe dieser Zustandsformen den gesamten $SO_2$-Gehalt in der Messprobe. Durch Ansäuerung wird das chemische Gleichgewicht zugunsten von $SO_2$ verschoben, welches dadurch im dynamischen chemischen Gleichgewicht aus der Probe durch Wasserdampfdestillation ausgetrieben werden kann. Im Weiteren bindet $SO_2$ mittels Bisulfit-Addition an Aldehyde und Ketone wie z.B. Acetaldehyd, dessen Rückreaktion durch Absenkung des pH-Werts ebenfalls begünstigt wird. D.h. je höher die Säurekonzentration in der Lösung ist, desto leichter erfolgt die Umsetzung des gebundenen $SO_2$ in gelöstes $SO_2$.

Vorzugsweise wird hierbei Phosphorsäure, insbesondere ortho-Phosphorsäure, verwendet. Die Handhabung von Phosphorsäure ist vorteilhaft aufgrund der hohen Verdampfungstemperatur. Noch bevorzugter wird für eine $SO_2$-Gehaltsbestimmung in Bier und Wein zur Ansäuerung eine Mischung aus Methanol, Wasser und ortho-Phosphorsäure eingesetzt.

Die Phosphorsäure liegt bevorzugt in einer Konzentration von 0.01 bis 5 mol/l, noch bevorzugter von 0.1 bis 2 mol/l, am meisten bevorzugt von 0.5 bis 1 mol/l vor.

Für Lebensmittel, bei welchen das $SO_2$ in besonderem Masse an dessen Inhaltsstoffen gebunden ist wie z.B. bei Trockenfrüchten, kann Schwefelsäure verwendet werden. Als Referenzprobe für die Analyse von Trockenfrüchten kann hierbei Hydroxymethylsulfonat eingesetzt werden. Die Schwefelsäure liegt bevorzugt in einer Konzentration von 0.02 bis 18 mol/l, noch bevorzugter von 3 bis 16 mol/l, am meisten bevorzugt von 8 bis 15 mol/l vor.

Wein enthält diverse flüchtige Säuren, welche bei einer Wasserdampfdestillation ebenfalls ins Auffanggefäss überführt werden. Eine Gehaltsbestimmung mittels Säure-Base-Titration anstelle der nachstehenden genauer beschriebenen iodometrischen Methode wäre dadurch mit einem Fehler behaftet. Trockenfrüchte hingegen besitzen nur einen geringen Gehalt an flüchtigen Säuren, weshalb bei solchen Proben alternativ die bei der Wasserdampfdestillation aufgefangene schweflige Säure z.B. mittels Wasserstoffperoxid zu Schwefelsäure umgesetzt und mittels Natronlauge titriert werden kann.

Vorzugsweise wird das $SO_2$ mittels Destillation, insbesondere Wasserdampfdestillation, in ein erstes Absorptionsgefäss überführt und mittels in Wasser gelösten, elementaren Iods zu Iodwasserstoff und Schwefelsäure überführt gemäss folgender Reaktion (i) :

$$SO_2 + I_2 + 4H_2O \rightarrow 2HI + SO_4^{2-} + 2H_3O^+ \qquad (i)$$

Das elementare Iod liegt zu Beginn der Messung in Wasser gelöst im ersten Absorptionsgefäss im Überschuss vor. Es lässt sich nicht ganz ausschliessen, dass das Iod aus dem ersten Absorptionsgefäss entweicht. Im Rahmen der vorliegenden Erfindung wird das während der Wasserdampfdestillation aus dem ersten Auffanggefäss entwichene Iod in geringen Mengen in einem zweiten Ethanol enthaltenden Auffanggefäss aufgefangen. Im zweiten Gefäss wird das Iod aufgrund der guten Löslichkeit in Ethanol festgehalten, welches damit als Absorptionsmittel fungiert. Diese Anordnung mit einem zweiten Absorptionsgefäss ist wichtig, um mögliche Iodverluste einzuschränken. Bei der Wasserdampfdestillation strömt ein Gasstrom aus Wasserdampf und Luft durch die Flüssigkeiten in den beiden Absorptionsgefässen. Dieser Gasstrom trägt Iodmoleküle mit sich. Mit dem Ethanol im zweiten Absorptionsgefäss wird das Iod abgefangen und der Verlust klein gehalten.

Das im zweiten Absorptionsgefäss aufgefangene gelöste Iod wird nach Abschluss der Destillation zur Rücktitration mit dem ersten Absorptionsgefäss vereinigt.

Die Rücktitration des elementaren Iods der vereinigten Absorptionsgefässe mit Thiosulfat erfolgt nach folgender Reaktion

(ii):

$$I_2 + 2S_2O_3^{2-} \rightarrow 2I^- + S_4O_6^{2-} \qquad \text{(ii)}$$

Das gelöste Iod wird zunächst ins erste Absorptionsgefäss bevorzugt in einer Konzentration von 0.001 bis 0.5 mol/l, noch bevorzugter von 0.01 bis 0.5 mol/l, am meisten bevorzugt von 0.01 bis 0.1 mol/l, zugegeben.

Abhängig von der Ausgestaltung einer Messvorrichtung und den Eigenschaften der eingesetzten Reagenzien kann auch die Messung einer Blindprobe zur Ermittlung eines geringen Messwertes führen, obwohl ein Vorhandensein der zu bestimmenden Substanz in der Blindprobe ausgeschlossen werden kann. Solche Blindwerte sind in erster Linie darauf zurück zu führen, dass ein Verlust von Iod aus dem oder den Absorptionsgefässen nie ganz ausgeschlossen werden kann. Blindwerte können aber auch entstehen, falls verunreinigte Reagenzien geringe Anteile der zu bestimmenden Substanz enthalten.

Aus diesen Gründen ist es wichtig, eine Blindwertbestimmung bei identischer Durchführung des erfindungsgemässen Verfahrens und vorgängig zur Durchführung des Verfahrens mit Referenzproben und Messproben vorzunehmen. Die ermittelten Blindwerte können dann bei der Kalibrationsgleichung berücksichtigt werden. Der Blindwert wird aufgrund des Gesamtgehalts des gelösten Iods ohne die Gegenwart von $SO_2$ ermittelt. Der Blindwert abzüglich dem Gehalt aus Reaktion (ii) dient zur Ermittlung des Iodanteils, welcher nicht durch $SO_2$ gemäss der Reaktion (i) zu Iodwasserstoff umgesetzt wurde.

Als Blindprobe wird bei der Analyse von Wein vorzugsweise eine Ethanollösung, insbesondere eine 12%-ige Ethanollösung, eingesetzt. Hierdurch besitzt die Blindprobe vorteilhafterweise einen vergleichbaren Ethanolgehalt wie Wein. Bei der Analyse von anderen Proben können entsprechend Blindproben verwendet werden, die der Probe möglichst ähnlich sind.

Ein weiterer Aspekt der Erfindung bezieht sich auf eine Vorrichtung zur Durchführung des erfindungsgemässen Verfahrens. Die Vorrichtung weist mindestens eine Destilliereinheit, mindestens ein Absorptionsgefäss, Mittel zur Gehaltsbestimmung in Form eines Titrators oder einer Bürette sowie Mittel zur Gehaltsberechnung auf, die dazu geeignet sind, die wahren Konzentrationswerte mit einer Kalibrationsgleichung zu erstellen. Typischerweise werden diese Mittel durch eine Softwaredatei gebildet, die mit einem herkömmlichen Rechner, insbesondere mittels einer Kalkulationssoftware, beispielsweise Microsoft Excel, lesbar ist.

Diese Vorrichtung kann im Weiteren bevorzugt zwei Absorptionsgefässe beinhalten, wobei im ersten Absorptionsgefäss das Destillat aufgefangen wird. Ein zweites mit dem ersten Gefäss möglichst luftdicht verbundenes Absorptionsgefäss dient dazu, die unreagierten Ioddämpfe aus dem ersten Absorptionsgefäss aufzunehmen.

Bei Einsatz von Iod gemäss der bevorzugten Ausführungsform wird $SO_2$ im ersten Absorptionsgefäss aufgefangen und reagiert mit diesem.

Ein Absorptionsmedium im zweiten Absorptionsgefäss muss einerseits die Eigenschaft haben, entwichenes Iod aus dem ersten Absorptionsgefäss aufzufangen beziehungsweise dieses gelöst in der Reaktionslösung vorliegende Iod zu fixieren. Andererseits darf dieses Absorptionsmedium die Reaktion des entwichenen Iods mit Thiosulfat und damit die Rücktitration nicht behindern. Es hat sich herausgestellt, dass sich als Absorptionsmedium Ethanol besonders gut eignet.

Die Erfindung wird nachfolgend in Ausführungsbeispielen und anhand der Zeichnungen näher erläutert. Es zeigen:

Figur 1:     eine schematische Darstellung einer erfindungsgemässen Vorrichtung

Figur 2:     eine schematische Darstellung von zwei Absorptionsgefässen einer Vorrichtung zur Durchführung des erfindungsgemässen Messverfahrens,

Figur 3:     eine grafische Darstellung einer linearen Korrelationsgleichung zwischen Einwaage und ermittelten $SO_2$-Gehalten der Referenzproben, und

Figur 4:     ein Fliessschema der Verfahrensschritte zur Durchführung des erfindungsgemässen Messverfahrens

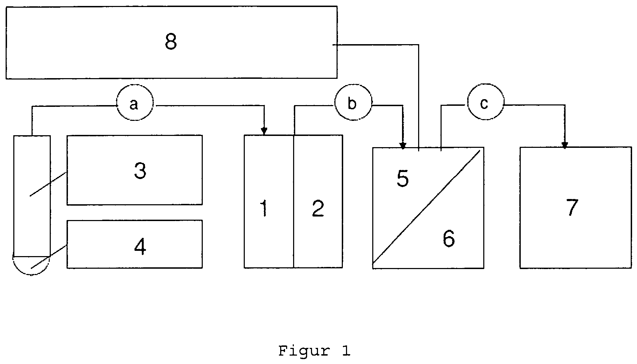

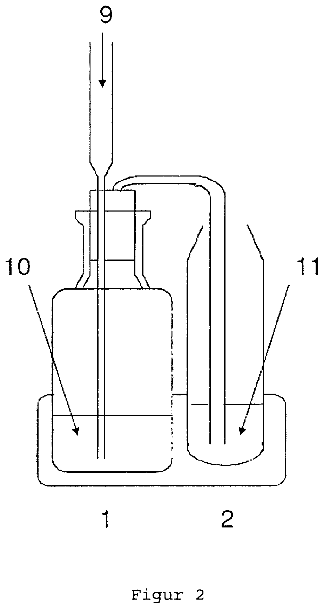

Figur 1 zeigt eine Vorrichtung zur $SO_2$-Gehaltsbestimmung in Wein oder Bier. Die Vorrichtung weist ein Gerät zur Wasserdampfdestillation 8 wie z.B. K-355 der Firma Büchi auf. Im Weiteren zeigt die Vorrichtung in Figur 1 ein Probenglas 3, in welches eine Probe 4 hineingegeben wird. Die Probe 4 wird hierbei durch eine Mischung aus Methanol, Wasser und 85%-ige ortho-Phosphorsäure im Verhältnis 500:400:50 versetzt. Eine Leitung a ist gezeigt, womit das aus der Probe 4 im Probenglas 3 entfernte Destillat 9 (Siehe Figur 2) mit $SO_2$ in ein erstes Absorptionsgefäss 1 überführt wird, das eine bekannte, überschüssige Menge an gelöstem, elementarem Iod 10 (Siehe Figur 2) in einer Konzentration von 0.05 mol/l enthält. Die 85%-ige ortho-Phosphorsäure stammt von der Firma Riedel-deHaen (30417). Das Methanol wird von der Firma Sigma Aldrich (32213) bezogen.

Das verwendete gelöste, elementare Iod 10 wird dabei als Iod-Standardlösung der Firma Riedel-deHaen (35090) bezogen. Im Weiteren zeigt die Vorrichtung gemäss Figur 2 ein zweites Absorptionsgefäss 2 zum Auffangen entwichener Ioddämpfe. Eine Leitung b ist im Weiteren in Figur 1 gezeigt, welche die vereinigten Absorptionsgefässe in einen Titrator 5 oder eine Bürette 6 überführt. Zudem weist die Vorrichtung gemäss Figur 1 schematisch mit Buchstaben c dargestellt Übertragungsmittel an ein Mittel zur Gehaltsberechnung 7 auf.

Figur 2 zeigt ein erstes Absorptionsgefäss 1, welches gelöstes elementares Iod enthält und in welches das Destillat 9 geleitet wird. Entwichenes, elementares Iod 10 wird gemäss Figur 2 in ein zweites Absorptionsgefäss 2 übergeleitet, welches vorzugsweise reines Ethanol 11, Ethanol purum, Fluka (02875), als Absorptionsmedium enthält.

Das überschüssige elementare Iod 10 im zweiten Absorptionsgefäss 2 wird nach Beendigung der Destillation mit dem Inhalt des ersten Absorptionsgefässes 1 vereinigt und die nun vereinigten Iodlösungen 10 beider Absorptionsgefässe werden mittels einer Lösung Natriumthiosulfat rücktitriert, vorzugsweise in einer Konzentration $c_T(Na_2S_2O_3)$ von 0.01 mmol/ml, gemäss Reaktion (ii). Diese Lösung Natriumthiosulfat wird von der Firma Riedel-deHaen (383243) bezogen.

Die Rücktitration gemäss Reaktion (ii) wird vorzugsweise mit einem Titrator 5 - wie z.B. Methrohm DMP 785 Titrino - als Mittel zur Gehaltsbestimmung ausgeführt, in welchen die vereinigte Lösung - wie schematisch mit dem Buchstaben b in Figur 1 dargestellt - überführt wird. Es handelt sich um eine Redoxreaktion, welche mit einer Redoxelektrode verfolgt werden kann und zur Ermittlung des Endpunkts der Titration eingesetzt wird. Alternativ bietet sich die Titration unter Zuhilfenahmen einer Bürette 6 und der Verwendung von heiss filtrierter Kartoffelstärke als Indikator. Anschliessend wird zur vollständigen Entfärbung von dunkelblau nach farblos titriert.

Aus der verbrauchten Menge Natriumthiosulfat gemäss der Reaktion (ii) kann letztlich indirekt der $SO_2$-Gehalt in der Probe 4 errechnet werden. Hierzu werden - wie schematisch mit dem Buchstaben c in Figur 1 dargestellt - die ermittelten Werte anhand des Titrators 5 beziehungsweise Bürette 6 durch ein Mittel zur Gehaltsberechnung 7 ausgewertet.

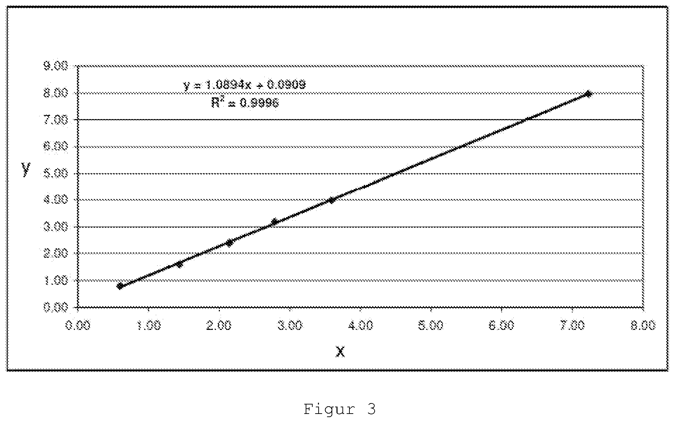

Wie in Figur 3 gezeigt werden unterschiedliche Einwaagen von Referenzproben mit bekanntem $SO_2$-Gehalt (y) und die mittels Rücktitration anhand der Vorrichtung gemäss Figur 1 ermittelten $SO_2$-Gehalte (x) bei identischen Messbedingungen (insbesondere Destillationsdauer) mittels linearer Regression rechnerisch in Beziehung gebracht. Eine lineare Korrelationgleichung y = f(x) wird erhalten.

Bei der vorgängigen Ermittlung der Korrelationsgleichung sollen die Referenzproben mit bekanntem $SO_2$-Gehalt in einer künstlichen Matrix stabilisiert sein. Diese stabilisierte Standardlösung wird erhalten, indem 120ml Ethanol, 323mg $Na_2SO_3$, 2.0267g Zitronensäuremonohydrat und 320ml Acetaldehydlösung in einen 1l Messkolben gegeben werden und mit destilliertem Wasser bis zu Marke aufgefüllt wird. Die Acetaldehydlösung wird durch Pipettieren von 1 bis 1.3ml Acetaldehyd (Ethanal, Fluka (00070)) in einen 1 Liter Messkolben mit 100ml deionisiertem Wasser erhalten.

Diese beschriebene Konzentration an Ethanol in der stabilisierten Standardlösung imitiert dessen ungefähren Gehalt im Wein. Anhand der zugegebenen Zitronensäure wird der pH-Wert der stabilisierten Standardlösung auf einen mit Wein vergleichbaren pH-Wert eingestellt. Insbesondere ist dies wichtig, um das chemische Gleichgewicht der unter den zusammengefassten Begriff $SO_2$ vorliegenden Produkte vergleichbar mit Wein zu simulieren. Im Weiteren wird durch die beschriebene Zugabe an Acetaldehyd in der stabilisierten Standardlösung vorteilhafterweise ebenfalls dessen ungefährer Gehalt in Wein imitiert, denn damit erfolgt anhand der Kalibrationsgleichung ebenfalls eine Korrektur des Fehlers aufgrund einer allenfalls unvollständigen Bisulfit-Rückreaktion durch Ansäuerung.

Jedes zugegebene Volumen der $SO_2$-Standardlösung entspricht einem bekannten Gehalt an $SO_2$. Es werden dabei mindestens fünf $SO_2$-Bestimmungen mit der 160 ppm $SO_2$-Standardlösung durchgeführt, um eine lineare Regressionsgerade zu erstellen. Dazu werden Volumina von 5 bis 50ml $SO_2$-Standardlösung zu den Probengläsern 3 pipettiert, z.B. 5, 10, 20, 30, 50ml. Optional können noch weitere Volumina von 15 bzw. 25ml pipettiert werden. Dadurch kann die Beziehung des $SO_2$-Gehalts der Standardlösungen und der ermittelten $SO_2$-Gehalte zu einer bestimmten Destillationsdauer exakt wiedergegeben werden, womit bei der anschliessenden Bestimmung der Messproben und Umrechnung gute Resultate zum $SO_2$-Gehalt in der Messprobe garantiert werden.

Ausserdem wird vorab eine Bestimmung des Blindwertes für den Verbrauch an Thiosulfat Standardlösung durchgeführt. Vor der Durchführung der Destillationen zur Ermittlung des Blindwerts wird vorzugsweise eine Konditionierung der erfindungsgemässen Vorrichtung, insbesondere des Geräts zur Wasserdampfdestillation mittels einer Destillation von 50ml Methanol während einer Destillationszeit von 5 bis 6 Minuten vorgenommen. Eine solche Konditionierung insbesondere des Geräts zur Wasserdampfdestillation mit Methanol ist vorteilhaft, um bei der Titration des verbleibenden Iods mit Thiosulfat Standardlösung konstante und stabile Resultate zu erhalten. Als Ziel sollten darauf die drei zuletzt ermittelten Blindwerte eine vorher spezifizierte relative Standardabweichung nicht überschreiten. Typischerweise werden jedoch bereits nach der Konditionierung mit Methanol stabile Blindwerte erzielt, so dass die drei folgenden Blindwerte zur Mittelwertbestimmung verwendet werden können. Die Konditionierung insbesondere des Geräts zur Wasserdampfdestillation mit Methanol entspricht etwa drei Destillationen mit der Mischung aus Methanol, Wasser und 85%iger ortho-Phosphorsäure im Verhältnis 500:400:50. Der erhaltene Mittelwert entspricht dem Gesamtgehalt beziehungsweise dem maximal aus der Vorrichtung gemäss Figur 1 messbaren Gehalt, d.h. der Umsetzung des gelösten Iods 10 ohne die Gegenwart von $SO_2$.

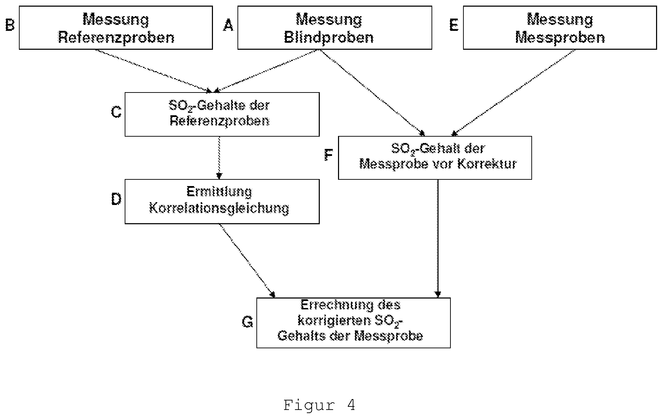

Das Fliessschema gemäss Figur 4 zeigt schematisch die Verfahrensschritte zur Durchführung des erfindungsgemässen Messverfahrens. Zunächst wird in einem Schritt A ein gemittelter Blindwert aus drei reproduzierbaren Blindwerten mit spezifizierter Standardabweichung ermittelt.

Anschliessend werden in einem Schritt B mehrere Volumina der Referenzproben zur Analyse in das Probenglas 3 der Vorrichtung gemäss Figur 1 gegeben unter identischen Messbedingungen beziehungsweise Destillationszeiten wie für die Blindproben in Schritt A. Das aufgefangene Destillat der Referenzproben wird zunächst im ersten Absorptionsgefäss aufgefangen und das enthaltene $SO_2$ gemäss Reaktion (i) umgesetzt. Anschliessend werden das erste Absorptionsgefäss 1 und zweite Absorptionsgefäss 2 vereinigt, wobei die vereinigte Lösung eine Restmenge an gelöstem Iod 10 enthält, welches nicht gemäss Reaktion (i) umgesetzt wurde. Gemäss Schritt C der Figur 4 sowie nachfolgend in einem Rechenbeispiel anhand der Gleichung (iii) numerisch erläutert, ergibt sich der messbare $SO_2$-Gehalt der Referenzproben aus dem vorgängig gemittelten Blindwert, abzüglich einem Differenzgehalt aus dem unreagierten Restgehalt an Iod 10, basierend auf dem titrierten Volumen der vereinigten Lösungen bei der Analyse der Referenzproben. Insbesondere wird das Differenzvolumen an titrierter Natriumthiosulfatlösung für die Blindproben und Referenzproben mit der Konzentration der Natriumthiosulfatlösung multipliziert, um die Molzahl an Natriumthiosulfat zu erhalten. Wie aus den Reaktionen (i) und (ii) ersichtlich, ist das Molverhältnis von Thiosulfat zu elementarem Iod beziehungsweise $SO_2$ 2:1, weshalb diese Molzahl noch halbiert werden muss. Anschliessend wird die erhaltene Molzahl mit dem Molekulargewicht von $SO_2$ multipliziert. Gemäss Schritt D der Figur 4 und numerisch basierend auf Werten der Tabelle 1 im nachfolgenden Rechenbeipiels erläutert, wird aufgrund der bekannten $SO_2$-Gehalte der Referenzproben (y) und der daraus ermittelten $SO_2$-Gehalte (x) eine Korrelationsgleichung gemäss Figur 3 ermittelt.

Unter identischen Messbedingungen beziehungsweise Destillationszeiten wie für die Blindproben beziehungsweise Referenzproben werden die Messproben in der Vorrichtung gemäss Figur 1 analysiert gemäss Schritt E in Figur 4.

Nach Schritt F (in Analogie zu Schritt C) der Figur 4 ergibt sich - wie anhand der Gleichung (iv) des nachfolgenden Rechenbeispiels numerisch erläutert - der messbare $SO_2$-Gehalt der Messproben aus dem vorgängig gemittelten Blindwert abzüglich einem Differenzgehalt aus dem unreagierten Restgehalt an Iod 10, basierend auf dem titrierten Volumen der vereinigten Lösungen bei der Analyse der Messproben.

Der hierbei erhaltene $SO_2$-Gehalt der Messprobe vor Korrektur beinhaltet einen Fehler insbesondere aufgrund der verkürzten Mess- bzw. Destillationszeit.

In Schritt G der Figur 4 wird der $SO_2$-Gehalt der Messprobe vor Korrektur deshalb - wie im nachfolgenden Rechenbeispiel numerisch erläutert - anhand der in Schritt D ermittelten Korrelationsgleichung korrigiert.

Die bevorzugte Ausführungsform beziehungsweise die Verfahrensschritte gemäss dem Fliessschema der Figur 4 werden anhand des nachfolgenden Rechenbeispiels erläutert.

Als Grundlage des Rechenbeispiels werden das Molekulargewicht von $SO_2$, $M(SO_2)$, die Konzentration der Lösung Natriumthiosulfat, $c_T(Na_2S_2O_3)$ sowie die Redoxvalenz $z_{Redoxvalenz\ von\ Thiosulfat}$ basierend auf Reaktion (ii) verwendet:

$c_T(Na_2S_2O_3)$ [mmol/ml] :  0.01

$M(SO_2)$ [mg/mmol]:  64.0648

$z_{Redoxvalenz\ von\ Thiosulfat}$ :  0.5

Im Weiteren werden im Rechenbeispiel folgende Begriffe verwendet:

$W(SO_2)_{Standard}$:  Berechneter $SO_2$-Gehalt basierend auf $V_{Standard}$

$W(SO_2)$:  Ermittelter $SO_2$-Gehalt der Referenzprobe

$W(SO_2)_{exp}$:  $SO_2$-Gehalt der Messprobe vor Korrektur

$W(SO_2)_{adj}$:  Korrigierter $SO_2$-Gehalt der Messprobe

$V_{Standard}$:  Volumen in ml der stabilisierten $SO_2$-Standardlösung

$V^T_{Blindprobe}$:  Verbrauch in ml an Natriumthiosulfatlösung (0.01mmol/ml) für die Messung der Blindprobe

$V^T_{Referenzprobe}$:  Verbrauch in ml an Natriumthiosulfatlösung (0.01mmol/ml) für die Messung der Referenzprobe

$V^T_{Messprobe}$:  Verbrauch in ml an Natriumthiosulfatlösung (0.01mmol/ml) für die Messung der Messprobe

Gemäss dem in Figur 4 beschriebenen Fliessschema werden in Schiritt B die Referenzproben als Volumina von 5, 10, 15, 20, 25 und 50ml der beschriebenen $SO_2$-Standardlösung $V_{Standard}$ auf den messbaren $SO_2$-Gehalt anhand des erfindungsgemässen Verfahrens untersucht. Gemäss Schritt A in Figur 4 werden vorgängig zur Messung mit den Referenzproben die Blindproben analysiert.

Für die Ermittlung der Masse beziehungsweise des Gehalt an $SO_2$ in einer Referenzprobe $W(SO_2)$ gemäss Schritt C in Figur bei Anwendung des Geräts zur Wasserdampfdestillation 8 Büchi K-355 der Vorrichtung gemäss Figur 1 gilt die Gleichung (iii):

$$W(SO_2) = (V^T_{Blindprobe} - V^T_{Referenzprobe}) \times c_T(Na_2S_2O_3) \times z \times M(SO_2) \quad (iii)$$

Folgende Tabelle 1 wird hierbei ermittelt:

Tabelle 1

| $V_{Standard}$ [ml] | $W(SO_2)$ K-355 [mg] (x) | $W(SO_2)_{Standard}$ [mg] (y) |
|---|---|---|
| 5 | 0.60 | 0.80 |
| 10 | 1.44 | 1.59 |
| 15 | 2.15 | 2.39 |
| 20 | 2.78 | 3.19 |
| 25 | 3.59 | 3.99 |
| 50 | 7.23 | 7.97 |

Gemäss Figur 3 sowie Schritt D werden die mit dem Verfahren mittels Rücktitration mit Natriumthiosulfat gemessenen $SO_2$-Gehalte der Referenzproben (x) und die bekannten $SO_2$-Gehalte der Referenzproben (y) grafisch aufgetragen. Anschliessend wird zwischen die Messpunkte mittels linearer Regression eine Gerade gelegt und die Korrelationsgleichung y = 1.0894x + 0.0909 ermittelt.

Für die Korrektur des $SO_2$-Gehalts der gemessenen $SO_2$-Gehalte der Messproben dient später die Steigung der linearen Korrelationsgleichung gemäss Figur 3, $a_{Steigung\ der\ Korrelationsgleichung}$: 1.0894.

Für jeweils eine Messprobe und Blindprobe mit einem Volumen von 50ml wird der $SO_2$-Gehalt ermittelt.

Mittels Rücktitration der vereinigten Iodlösungen 10 aus den beiden Absorptionsgefässen mit Natriumthiosulfat wurde bei der Verwendung von drei Blindproben folgender Blindwert gemittelt:

$$V^T_{Blindprobe} \text{ [ml]:} \quad 47.855 \text{ (Standardabweichung 0.375ml)}$$

Mittels Rücktitration der vereinigten Iodlösungen 10 aus den beiden Adsorptionsgefässen mit Thiosulfat-Standardlösung wurden bei einer Zweifachbestimmung folgender Messwert ermittelt gemäss Schritt E, Figur 4:

$$V^T_{Messprobe} \text{ [ml]:} \quad 35.096$$

Es gilt für $W(SO_2)_{exp}$ als der berechnete Gehalt beziehungsweise Masse $SO_2$ in der Messprobe vor der Korrektur mittels Korrelationsgleichung 9 gemäss Schritt F, Figur 4:

$$W(SO_2)_{exp} = (V^T_{Blindprobe} - V^T_{Messprobe}) \times c_T(Na_2S_2O_3) \times z \times M(SO_2) \quad (iv)$$
$$= (47.855 - 35.096) \times 0.01mmol/ml \times 0.5 \times 64.0648mg/mmol$$
$$= 4.087mg$$

Anschliessend wird anhand der Steigung der Korrelationsgleichung (a) der ermittelte Wert $W(SO_2)_{exp}$ auf den $SO_2$-Gehalt in der Messprobe 11 bzw. $W(SO_2)_{adj}$ umgerechnet wie in Schritt G, Figur 4 erläutert.

$$W(SO_2)_{adj} = a \times W(SO_2)_{exp}$$
$$= 1.0894 \times 4.087 = 4.452mg$$

**Patentansprüche**

1. Verfahren zur Bestimmung des $SO_2$-Gehalts in einer Probe (4) von Getränken und Lebensmitteln mit den Verfahrensschritten:

- Wasserdampfdestillation zum Abtrennen des $SO_2$ aus der Probe (4) und Auffangen des $SO_2$-haltigen Destillats (9)

- Gehaltsbestimmung des $SO_2$ der Probe (4) anhand des Destillats (9) zum Erzielen von $SO_2$-Messwerten

wobei die bestimmten $SO_2$-Messwerte der Probe (4) anhand einer mittels Referenzproben (7) mit bekanntem $SO_2$-Gehalt ermittelten Kalibrationsgleichung auf einen korrigierten $SO_2$-Gehalt der Messprobe (11) umgerechnet werden, wobei die Messbedingungen und die Destillationsdauer für die gemessenen $SO_2$-Gehalte aus den Referenzproben (7) mit bekannten $SO_2$-Gehalten zur Erstellung der Kalibrationsgleichung und die Messbedingungen zur Gehaltsbestimmung der Messproben (8) identisch sind,

**dadurch gekennzeichnet, dass**

- bei der Wasserdampfdestillation das Destillat (9) in eine Iodlösung (10) in einem ersten Absorptionsgefäss (1) geleitet wird, welche einen Überschuss an Iod (10) enthält so dass nach der Reaktion des $SO_2$ mit dem Iod (10) eine titrierbare Restmenge Iod (10) vorliegt;
- in einem zweiten Absorptionsgefäss (2) die durch den Destillationsprozess aus der Iodlösung (10) des ersten Absorptionsgefäss (1) entwichene Iodmenge (10) in Ethanol (11) abgefangen wird;
- und die Iodlösungen (10) des ersten Absorptionsgefässes (1) und zweiten Absorptionsgefässes (2) vereinigt und die Restmenge Iod (10) titrimetrisch insbesondere mit Thiosulfat in einer Redoxrückreaktion bestimmt wird.

2. Verfahren zur Bestimmung des $SO_2$-Gehalts in Getränken und Lebensmitteln gemäss Anspruch 1, **dadurch gekennzeichnet, dass** die Kalibrationsgleichung eine Korrelationsgleichung (9) ist, welche rechnerisch die ermittelten $SO_2$-Messwerte mit den bekannten $SO_2$-Gehalten in Beziehung setzt.

3. Verfahren zur Bestimmung des $SO_2$-Gehalts in Getränken und Lebensmitteln gemäss Anspruch 2, **dadurch gekennzeichnet, dass** die Korrelationsgleichung (9) eine lineare Gleichung ist, ermittelt anhand einer linearen Regressionsanalyse.

4. Verfahren zur Bestimmung des $SO_2$-Gehalts in Getränken und Lebensmitteln gemäss einem der vorherigen Ansprüchen, **dadurch gekennzeichnet, dass** vorgängig zur Destillation die Referenz- bzw. Messproben angesäuert werden.

5. Verfahren zur Bestimmung des $SO_2$-Gehalts in Getränken und Lebensmitteln gemäss Anspruch 4, **dadurch gekennzeichnet, dass** die Ansäuerung der Probe (4) mit Hilfe von Phosphorsäure erfolgt.

6. Verfahren zur Bestimmung des $SO_2$-Gehalts in Getränken und Lebensmitteln gemäss Anspruch 5, **dadurch gekennzeichnet, dass** die Phosphorsäure bevorzugt in einer Konzentration von 0.01 bis 5 mol/l, noch bevorzugter von 0.1 bis 2 mol/l, am meisten bevorzugt von 0.5 bis 1 mol/l vorliegt.

7. Verfahren zur Bestimmung des $SO_2$-Gehaltes in Getränken und Lebensmitteln gemäss Anspruch 6, **dadurch gekennzeichnet, dass** das elementare Iod als Lösung (10) bevorzugt in einer Konzentration von 0.001 bis 0.5 mol/l, noch bevorzugter von 0.01 bis 0.5 mol/l, am meisten bevorzugt von 0.01 bis 0.1 mol/l vorliegt.

8. Verfahren zur Bestimmung des $SO_2$-Gehalts in Getränken und Lebensmitteln gemäss einem der vorherigen Ansprüche, **dadurch gekennzeichnet, dass** eine Blindwertbestimmung vorgängig und unter identischen Messbedingungen wie für die Messproben und Referenzproben durchgeführt wird und die titrimetrisch ermittelte Restmenge Iod (10) der vereinigten Absorptionsgefässe aus den Bestimmungen der Referenzproben und Messproben vom Blindwert abgezogen werden.

9. Verfahren zur Bestimmung des $SO_2$-Gehalts in Getränken und Lebensmitteln gemäss Anspruch 8, **dadurch gekennzeichnet, dass** als Blindwert eine Ethanollösung eingesetzt wird.

10. Vorrichtung zur Durchführung des Verfahrens gemäss den vorherigen Ansprüchen enthaltend

- eine Destilliereinheit,
- mindestens ein Absorptionsgefäss,
- Mittel zur Gehaltsbestimmung ausgewählt aus einem Titrator (5) oder einer Bürette (6) und
- Mittel zur Gehaltsberechnung (7)

**dadurch gekennzeichnet, dass** das Mittel zur Gehaltsberechnung so gestaltet ist, dass der $SO_2$- Gehalt der Messprobe anhand einer ermittelten Korrelationsgleichung korrigiert wird, und dass

- die Vorrichtung ein erstes Absorptionsgefäss (1) zum Auffangen des $SO_2$-haltigen Destillats (9) in Iodlösung (10) und
- ein mit dem ersten Absorptionsgefäss (1) verbundenes zweites Absorptionsgefäss (2) zum Auffangen während des Destillationsprozesses entweichender Ioddämpfe aufweist.

**Claims**

1. Method for determining the $SO_2$ content in a sample (4) of drink and food with the method steps of:

   - steam distillation to separate the $SO_2$ from the sample (4) and collection of the $SO_2$-containing distillate (9)
   - determination of the $SO_2$ content of the sample (4) based on the distillate (9) to achieve $SO_2$ measurement values

   wherein the determined $SO_2$ measurement values of the sample (4) are converted, based on a calibration equation determined by means of reference samples (7) with known $SO_2$ content, to give a corrected $SO_2$ content of the test sample (11), , wherein the measurement conditions and the duration of distillation for the measured $SO_2$ contents of the reference samples (7) with known $SO_2$ contents for generating the calibration equation and the measurement conditions for the content determination of the test samples (8) are identical,
   **characterized in that**

   - in the steam distillation, the distillate (9) is passed into an iodine solution (10) in a first absorption vessel (1), which contains an excess of iodine (10), such that after the reaction of $SO_2$ and iodine (10) a residual amount of iodine (10) is present that can be titrated;
   - the amount of iodine (10) released from the iodine solution (10) of the first absorption vessel (1) by the distillation process is retained in ethanol (11) in a second absorption vessel (2);
   - and the iodine solutions (10) of the first absorption vessel (1) and the second absorption vessel (2) are combined and the residual amount of iodine (10) is determined by titration, in particular with thiosulfate, in a reverse redox reaction,.

2. Method for determining the $SO_2$ content in drinks and food according to Claim 1, **characterized in that** the calibration equation is a correlation equation (9) which mathematically relates the determined $SO_2$ measurement values with the known $SO_2$ contents.

3. Method for determining the $SO_2$ content in drinks and food according to Claim 2, **characterized in that** the correlation equation (9) is a linear equation, determined by means of a linear regression analysis.

4. Method for determining the $SO_2$ content in drinks and food according to any of the preceding claims, **characterized in that** the reference and test samples are acidified prior to distillation.

5. Method for determining the $SO_2$ content in drinks and food according to Claim 4, **characterized in that** the sample (4) is acidified with phosphoric acid.

6. Method for determining the $SO_2$ content in drinks and food according to Claim 5, **characterized in that** the phosphoric acid is present preferably at a concentration of 0.01 to 5 mol/l, more preferably 0.1 to 2 mol/l, most preferably 0.5 to 1 mol/l.

7. Method for determining the $SO_2$ content in drinks and food according to Claim 6, **characterized in that** the elemental iodine is present as a solution (10) preferably at a concentration of 0.001 to 0.5 mol/l, more preferably 0.01 to 0.5 mol/l, most preferably 0.01 to 0.1 mol/l.

8. Method for determining the $SO_2$ content in drinks and food according to any of the preceding claims, **characterized in that** a blank value determination is carried out in advance and under identical measurement conditions as for the test samples and reference samples, and the residual amount of iodine (10), determined by titration, of the combined absorption vessels from the determinations of the reference samples and test samples is deducted from the blank value.

9. Method for determining the SO$_2$ content in drinks and food according to Claim 8, **characterized in that** an ethanol solution is used as blank value.

10. Device for performing the method according to the preceding claims comprising

- a distillation unit,
- at least one absorption vessel,
- means for determining a content selected from a titrator (5) or a burette (6), and
- means for calculating a content (7)

**characterized in that** the means for calculating the content is adapted such that the SO$_2$ content of the test sample is corrected by means of a determined correlation equation, and **in that**

- the device comprises a first absorption vessel (1) for collecting the SO$_2$-containing distillate (9) in iodine solution (10) and
- a second absorption vessel (2) linked to the first absorption vessel (1) for collecting iodine vapour released during the distillation process.

**Revendications**

1. Procédé de détermination de la teneur en SO$_2$ dans un échantillon (4) de boissons et d'aliments, comprenant les étapes suivantes:

- distillation à la vapeur d'eau pour séparer le SO$_2$ de l'échantillon (4) et recueillir le distillat (9) contenant du SO$_2$,
- détermination de la teneur en SO$_2$ de l'échantillon (4) à l'aide du distillat (9) pour obtenir des valeurs de mesure de SO$_2$,

dans lequel les valeurs de mesure de SO$_2$ déterminées de l'échantillon (4) sont converties en une teneur en SO$_2$ corrigée de l'échantillon de mesure (11) à l'aide d'une équation de calibrage déterminée au moyen d'échantillons de référence (7) avec une teneur en SO$_2$ connue, dans lequel les conditions de mesure et la durée de distillation pour les teneurs en SO$_2$ mesurées à partir des échantillons de référence avec des teneurs en SO$_2$ connues pour établir l'équation de calibrage et les conditions de mesure pour la détermination de la teneur des échantillons de mesure (8) sont identiques, **caractérisé en ce que**

- lors de la distillation à la vapeur d'eau, le distillat (9) est conduit dans une solution d'iode (10) dans un premier récipient d'absorption (1), qui contient un excès d'iode (10), de telle manière qu'après la réaction du SO$_2$ avec l'iode (10) on obtienne une quantité résiduelle titrable d'iode (10) ;
- dans un deuxième récipient d'absorption (2), la quantité d'iode (10) dégagée par le processus de distillation à partir de la solution d'iode (10) du premier récipient d'absorption (1) est capturée dans l'éthanol (11) ;
- et les solutions d'iode (10) du premier récipient d'absorption (1) et du deuxième récipient d'absorption (2) sont réunies et la quantité résiduelle d'iode (10) est déterminée par titrimétrie en particulier avec du thiosulfate dans une réaction redox inverse.

2. Procédé de détermination de la teneur en SO$_2$ dans des boissons et des aliments selon la revendication 1, **caractérisé en ce que** l'équation de calibrage est une équation de corrélation, qui met en relation par le calcul les valeurs de mesure de SO$_2$ déterminées avec les teneurs en SO$_2$ connues.

3. Procédé de détermination de la teneur en SO$_2$ dans des boissons et des aliments selon la revendication 2, **caractérisé en ce que** l'équation de corrélation (9) est une équation linéaire, déterminée à l'aide d'une analyse de régression linéaire.

4. Procédé de détermination de la teneur en SO$_2$ dans des boissons et des aliments selon l'une quelconque des revendications précédentes, **caractérisé en ce que** l'on acidifie les échantillons de référence et de mesure avant la distillation.

**5.** Procédé de détermination de la teneur en $SO_2$ dans des boissons et des aliments selon la revendication 4, **caractérisé en ce que** l'on effectue l'acidification de l'échantillon (4) à l'aide d'acide phosphorique.

**6.** Procédé de détermination de la teneur en $SO_2$ dans des boissons et des aliments selon la revendication 5, **caractérisé en ce que** l'acide phosphorique se trouve de préférence à une concentration de 0,01 à 5 moles/litre, de préférence encore de 0,1 à 2 moles/litre, et de préférence encore de 0,5 à 1 mole/litre.

**7.** Procédé de détermination de la teneur en $SO_2$ dans des boissons et des aliments selon la revendication 6, **caractérisé en ce que** l'iode élémentaire se trouve sous forme de solution (10) de préférence à une concentration de 0,001 à 0,5 mole/litre, de préférence encore de 0,01 à 0,5 mole/litre, et de préférence encore de 0,01 à 0,1 mole/litre.

**8.** Procédé de détermination de la teneur en $SO_2$ dans des boissons et des aliments selon l'une quelconque des revendications précédentes, **caractérisé en ce que** une détermination à blanc est effectuée préalablement et sous conditions identiques à celles pour les échantillons de mesure et les échantillons de référence et la quantité résiduelle d'iode (10) des récipients d'absorption réunis déterminée par titrimétrie à partir des déterminations des échantillons de référence et des échantillons de mesure est déduite de la valeur à blanc.

**9.** Procédé de détermination de la teneur en $SO_2$ dans des boissons et des aliments selon la revendication 8, **caractérisé en ce que** une solution d'éthanol est utilisée comme valeur à blanc.

**10.** Dispositif pour la mise en oeuvre du procédé selon les revendications précédentes, contenant

- une unité de distillation,
- au moins un récipient d'absorption,
- un moyen pour déterminer les teneurs sélectionnés parmi un titreur (5) ou une burette (6), et
- un moyen pour calculer les teneurs (7),

**caractérisé en ce que** le moyen pour calculer les teneurs est adapté de telle manière que la teneur en $SO_2$ de l'échantillon de mesure soit corrigée à l'aide d'une équation de corrélation déterminée, et **en ce que** le dispositif présente

- un premier récipient d'absorption (1) pour recueillir le distillat (9) contenant du $SO_2$ dans une solution d'iode (10), et
- un deuxième récipient d'absorption (2) relié au premier récipient d'absorption (1) pour recueillir des vapeurs d'iode dégagées pendant le processus de distillation.

Figur 1

Figur 2

Figur 3

B | Messung
Referenzproben

A | Messung
Blindproben

E | Messung
Messproben

C | SO$_2$-Gehalte der
Referenzproben

F | SO$_2$-Gehalt der
Messprobe vor Korrektur

D | Ermittlung
Korrelationsgleichung

G | Errechnung des
korrigierten SO$_2$-
Gehalts der Messprobe

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0190742 A1